# EUROPÄISCHE PATENTANMELDUNG

(11) **EP 4 574 807 A1**
(43) Veröffentlichungstag der Anmeldung: **25.06.2025**
(21) Anmeldenummer: 23219604.8
(22) Anmeldetag: 22.12.2023
(51) Int. Cl.: C07C 29/132, C07C 31/125, C07C 31/20, C07C 33/22, C07C 41/28, C07C 43/13

(54) **VERFAHREN ZUR RUTHENIUM-KATALYSIERTEN HYDRIERUNG VON ALDEHYDACETALEN**

(71) Anmelder: Evonik Oxeno GmbH & Co. KG, 45772 Marl (DE)
(72) Erfinder: SCHAREINA, Thomas, 18195 Cammin (DE); SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45722 Marl (DE)
(74) Vertreter: Evonik Patent Association

(57) **Zusammenfassung**

Verfahren zur Ruthenium-katalysierten Hydrierung von Aldehydacetalen.

## Beschreibung

Die vorliegende Erfindung betrifft ein Verfahren zur Ruthenium-katalysierten Hydrierung von Aldehydacetalen.

In DE 42 20 939 A1 wird ein Verfahren zur Herstellung von 2-Aryl-ethanolen beschrieben:

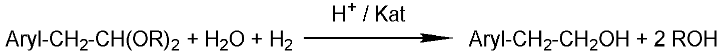

Die Reaktion erfolgt in einem schwefelsaurem Medium und wird durch einen Ru/C-Katalysator katalysiert, also einem Ru-Trägerkatalysator mit Kohlepulver als Träger.

Der vorliegenden Erfindung lag die Aufgabe zugrunde ein Verfahren zur Ruthenium-katalysierten Hydrierung von Aldehydacetalen bereitzustellen, mit welchen eine gute Ausbeute erzielt werden kann.

Diese Aufgabe wird gelöst durch ein Verfahren gemäß Anspruch 1.

Verfahren umfassend die Verfahrensschritte:
a) Vorlegen eines Aldehydacetals gemäß einer der Formeln (la) bis (VIa): 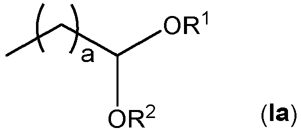 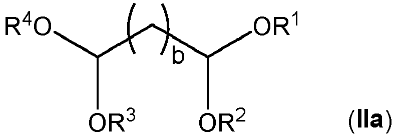 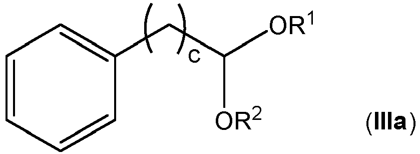 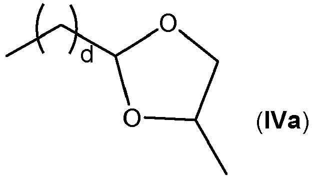 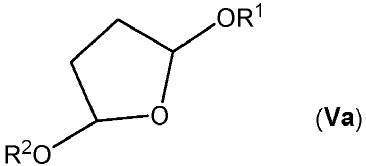 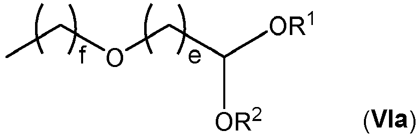
   wobei a, c, d, f für eine ganze Zahlen von 0 bis 12 stehen und b, e für eine ganze Zahlen von 1 bis 12 stehen,
   und R¹, R², R³, R⁴, jeweils unabhängig voneinander, für (C₁-C₁₂)-Alkyl stehen;
b) Zugabe einer Ru-Verbindung, welche zur Ausbildung eines Komplexes befähigt ist und eines Liganden, welcher ein P-Atom aufweist, oder
   eines Ru-Liganden-Komplexes, wobei der Ligand des Komplexes ein P-Atom aufweist;
c) Zuführen von H₂;
d) Erhitzen des Reaktionsgemisches aus a) bis c), wobei das Aldehydacetal zu einer Verbindung gemäß Formel (**Ib**) bis (**Vlb**) umgesetzt wird:

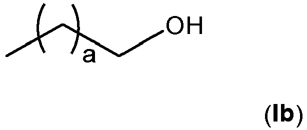

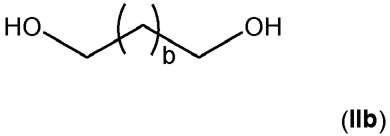

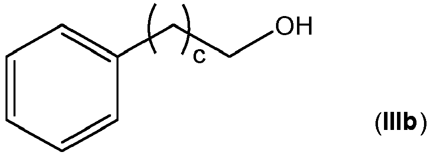

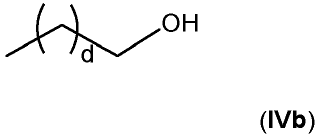

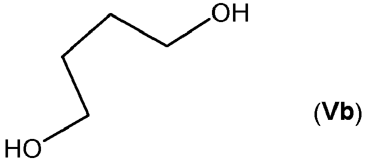

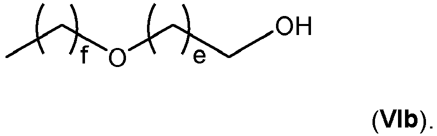

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

In einer Variante des Verfahrens stehen R¹, R² für den gleichen Rest.

In einer Variante des Verfahrens stehen R³, R⁴ für den gleichen Rest.

In einer Variante des Verfahrens stehen R¹, R², R³, R⁴ für den gleichen Rest.

In einer Variante des Verfahrens stehen R¹, R², R³, R⁴ für (C₁-C₄)-Alkyl.

In einer Variante des Verfahrens ist die Ru-Verbindung ausgewählt aus: RuCl₃ × 3H₂O, [Ru(Cymen)Cl₂]₂, RuBr₃ × 3H₂O, RuI₃, Ru(PPh₃)₃Cl₂.

In einer Variante des Verfahrens handelt es sich bei dem Liganden um eine Phosphinliganden oder Phosphitliganden.

In einer Variante des Verfahrens handelt es sich bei dem Liganden um eine Phosphinliganden.

In einer Variante des Verfahrens ist der Ligand ausgewählt aus: PPh₃, 1,4-Bis-(diphenylphosphino)-butan (dppb), 1,1'-Ferrocenediyl-bis(diphenylphosphin) (dppf), Bis-[2-(diphenylphosphino)-phenyl]-ether (dpephos), 1,3-Bis-(diphenylphosphino)-propan (dppp), 4,5-Bis-(diphenylphosphino)-9,9-dimethylxanthen (XantPhos).

In einer Variante des Verfahrens erfolgt das Zuführen von H₂ mit einem Druck im Bereich von 0,5 MPa (5 bar) bis 8 MPa (80 bar).

In einer Variante des Verfahrens erfolgt das Zuführen von H₂ mit einem Druck im Bereich von 1 MPa (10 bar) bis 6 MPa (60 bar).

In einer Variante des Verfahrens erfolgt das Erhitzen auf eine Temperatur im Bereich von 30 °C bis 100 °C.

In einer Variante des Verfahrens erfolgt das Erhitzen auf eine Temperatur im Bereich von 40 °C bis 80 °C.

In einer Variante des Verfahrens umfasst das Verfahren den zusätzlichen Verfahrensschritt c`): c') Zugabe eines Lösungsmittels.

In einer Variante des Verfahrens ist das Lösungsmittel ausgewählt aus: 1,4-Dioxan, Tetrahydrofuran (THF), Wasser.

Im Folgenden soll die Erfindung anhand von Ausführungsbeispielen näher erläutert werden.

### Versuchsbeschreibung

### Allgemein (Autoklav)

Ein 8-mL-Fläschchen wurde mit den entsprechenden Mengen an RuCl₃ × 3H₂O, PPh₃ und einem Magnetrührer gefüllt. Das Fläschchen wurde dann mit einem Septum (PTFE-beschichteter Silikonkautschuk) und einem Phenolharzdeckel verschlossen. Das Fläschchen wurde über eine Nadel mit der Argon-Zuleitung verbunden. Das Fläschchen wurde evakuiert und dreimal mit Argon aufgefüllt. Ein Ether-Lösungsmittel (THF oder 1,4-Dioxan, unter Argon gelagert) und die entsprechenden Mengen an Wasser und Substrat wurden mit einer Spritze in das Fläschchen injiziert, so dass eine dunkle Lösung entstand.

Das Fläschchen wurde in eine Edelstahlplatte gestellt, wobei die Nadel noch an Ort und Stelle verblieb, um den Gasaustausch im Autoklaven zu ermöglichen. Die Platte wurde in einen Autoklaven (300 mL) der Serie 4760 von Parr Instruments unter Argonatmosphäre überführt. Nach dreimaligem Spülen des Autoklaven mit Wasserstoff wurde der Wasserstoffdruck bei Raumtemperatur auf 20 bar / 40 bar erhöht. Die Reaktion wurde durch Erhitzen des Autoklaven in einem Aluminiumblock auf einem Heiz-/Rührgerät mit Magnetrühren für 18 h bei 60 °C (Temperatur des Aluminiumblocks) durchgeführt. Nach Ablauf der Reaktionszeit wurde der Autoklav auf Raumtemperatur abgekühlt und der Druck vorsichtig abgelassen. Dann wurde Tetradecan (0,100 mL) als interner Standard eingespritzt.

### A) 1,1-Dimethoxynonan zu 1-Nonanol

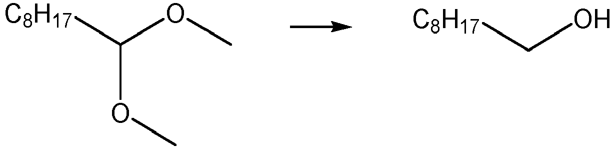

THF (1.5 mL), 0.135 mL (7.5 mmol) Wasser und 0.55 mL (0.47 g, 2.5 mmol) 1,1-Dimethoxynonane wurden zu 1,3 mg (0.2 mol%) RuCl₃ × 3H₂O und 5,2 mg (0.8 mol%) PPh₃ gegeben. Es werden 20 bar H₂ aufgepresst und die Reaktion 18 h bei 60 °C durchgeführt. Ausbeute (GC) quantitativ.

### Variation des Liganden

### 1,4-Bis-(diphenylphosphino)-butan (dppb):

Dioxan (1.5 mL, absolut), 0.36 mL (15 mmol) Wasser und 0.55 mL (0.47 g, 2.5 mmol) 1,1-Dimethoxynonane werden zu 2,6 mg (0.4 mol%) RuCl₃ × 3H₂O und 8.5 mg (0.04 mmol, 0.8 mol%) 1,4-Bis-(diphenylphosphino)-butan gegeben. Es werden 20 bar H₂ aufgepresst und die Reaktion 18 h bei 60 °C durchgeführt. Ausbeute (GC) quantitativ.

### 1,1'-Ferrocenediyl-bis(diphenylphosphin) (dppf):

Dioxan (1.5 mL, absolut), 0.36 mL (15 mmol) Wasser und 0.55 mL (0.47 g, 2.5 mmol) 1,1-Dimethoxynonane werden zu 2,6 mg (0.4 mol%) RuCl₃ × 3H₂O und 11.1 mg (0.04 mmol, 0.8 mol%) 1,1'-Ferrocenediyl-bis(diphenylphosphin) gegeben. Es werden 20 bar H₂ aufgepresst und die Reaktion 18 h bei 60 °C durchgeführt. Ausbeute (GC) quantitativ.

### Bis-[2-(diphenylphosphino)-phenyl]-ether (dpephos):

Dioxan (1.5 mL, absolut), 0.36 mL (15 mmol) Wasser und 0.55 mL (0.47 g, 2.5 mmol) 1,1-Dimethoxynonane werden zu 2,6 mg (0.4 mol%) RuCl₃ × 3H₂O und 10.8 mg (0.04 mmol, 0.8 mol% Bis-[2-(diphenylphosphino)-phenyl]-ether gegeben. Es werden 20 bar H₂ aufgepresst und die Reaktion 18 h bei 60 °C durchgeführt. Ausbeute (GC) quantitativ.

### 1,3-Bis-(diphenylphosphino)-propan (dppp):

Dioxan (1.5 mL, absolut), 0.36 mL (15 mmol) Wasser und 0.55 mL (0.47 g, 2.5 mmol) 1,1-Dimethoxynonane werden zu 2,6 mg (0.4 mol%) RuCl₃ × 3H₂O und 8.2 mg (0.04 mmol, 0.8 mol% 1,3-Bis-(diphenylphosphino)-propan gegeben. Es werden 20 bar H₂ aufgepresst und die Reaktion 18 h bei 60 °C durchgeführt. Ausbeute (GC) quantitativ.

### 4,5-Bis-(diphenylphosphino)-9,9-dimethylxanthen (XantPhos):

Dioxan (1.5 mL, absolut), 0.36 mL (15 mmol) Wasser und 0.55 mL (0.47 g, 2.5 mmol) 1,1-Dimethoxynonane werden zu 2,6 mg (0.4 mol%) RuCl₃ × 3H₂O und 11.6 mg (0.02 mmol, 0.8 mol% 4,5-Bis-(diphenylphosphino)-9,9-dimethylxanthen gegeben. Es werden 20 bar H₂ aufgepresst und die Reaktion 18 h bei 60 °C durchgeführt. Ausbeute (GC) 59%.

### Variation der Ru-Verbindung

### [Ru(Cymen)Cl₂]₂:

Dioxan (1.5 mL, absolut), 0.36 mL (15 mmol) Wasser und 0.55 mL (0.47 g, 2.5 mmol) 1,1-Dimethoxynonan werden zu 3.1 mg (0.4 mol% Ruthenium) [Ru(Cymen)Cl₂]₂ und 10.5 mg (0.04 mmol, 1.6 mol%) PPh₃ gegeben. Es werden 20 bar H₂ aufgepresst und die Reaktion 18 h bei 60 °C durchgeführt. Ausbeute (GC) quantitativ.

### RuBr₃ × 3H₂O:

Dioxan (1.5 mL, absolut), 0.36 mL (15 mmol) Wasser und 0.55 mL (0.47 g, 2.5 mmol) 1,1-Dimethoxynonan werden zu 3.9 mg (0.4 mol% Ruthenium) RuBr₃ × 3H₂O und 10.5 mg (0.04 mmol, 1.6 mol%) PPh₃ gegeben. Es werden 20 bar H₂ aufgepresst und die Reaktion 18 h bei 60 °C durchgeführt. Ausbeute (GC) 93%.

### RuI₃:

Dioxan (1.5 mL, absolut), 0.36 mL (15 mmol) Wasser und 0.55 mL (0.47 g, 2.5 mmol) 1,1-Dimethoxynonan (0.47 g, 2.5 mmol) werden zu 4.8 mg (0.4 mol% Ruthenium) RuI₃ und 10.5 mg (0.04 mmol, 1.6 mol%) PPh₃ gegeben. Es werden 20 bar H₂ aufgepresst und die Reaktion 18 h bei 60 °C durchgeführt. Ausbeute (GC) 75%.

### Ru(PPh₃)₃Cl₂:

Dioxan (1.5 mL, absolut), 0.36 mL (15 mmol) Wasser und 0.55 mL (0.47 g, 2.5 mmol) 1,1-Dimethoxynonan werden zu 9.6 mg (0.4 mol% Ruthenium) RuBr₃ × 3H₂O und 2.6 mg (0.01 mmol, 0.4 mol%) PPh₃ gegeben. Es werden 20 bar H₂ aufgepresst und die Reaktion 18 h bei 60 °C durchgeführt. Ausbeute (GC) quantitativ.

### Variation des Aldehydacetals

### B) 1,1,6,6-Tetramethoxyhexan zu 1,6-Hexandiol

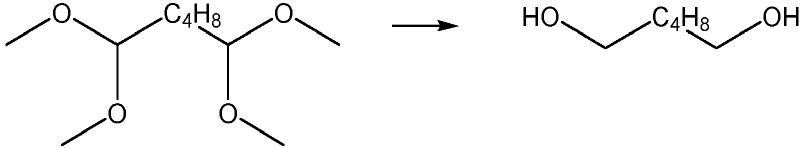

1,4-Dioxan (6 mL, absolut), 1.44 mL (80 mmol) Wasser und 2,06 g (10 mmol) 1,1,6,6-Tetramethoxyhexan,10.5 mg (0.4 mol%) RuCl₃ × 3H₂O und 42 mg (1.6 mol%) PPh₃ werden in ein Vial gegeben. Es werden 40 bar H₂ aufgepresst und die Reaktion 18 h bei 60 °C durchgeführt. Die GC-Ausbeute beträgt 81%.

### C) 1,1,6,6-Tetrabutoxyhexan zu 1,6-Hexandiol

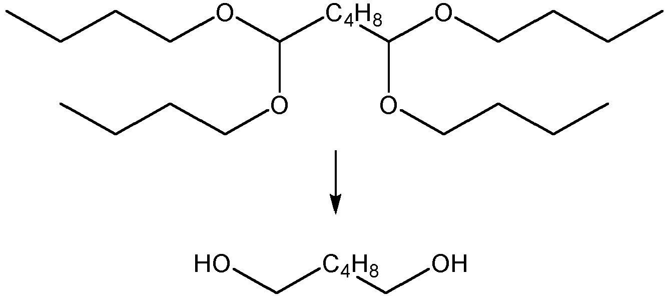

1.5 mL THF, 0.99 g (2,64 mmol) 1,1,6,6-Tetrabutoxyhexan und 0.72 mL H₂O, 2.6 mg (0.38 mol%) RuCl₃ × 3H₂O und 10.5 mg (1.52 mol%) PPh₃. in ein Vial gegeben. Es werden 20 bar H₂ aufgepresst und die Reaktion 18 h bei 60 °C durchgeführt. Die GC-Ausbeute beträgt 90%.

### D) 1,1,4,4-Tetramethoxybutan zu 1,4-Butandiol

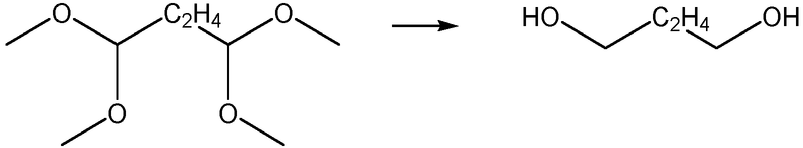

1.5 mL Dioxan, 0.54 mL Wasser und 0,46 g (2.56 mmol) 1,1,4,4-Tetramethoxybutan, 2.6 mg (0.39 mol%) RuCl₃ × 3H₂O und 10.5 mg (1.56 mol%) PPh₃ werden in ein Vial gegeben. Es werden 20 bar H₂ aufgepresst und die Reaktion 18 h bei 60 °C durchgeführt. Die NMR-Ausbeute beträgt 66%.

### E) Benzaldehyddimethylacetal zu Benzylalkohol

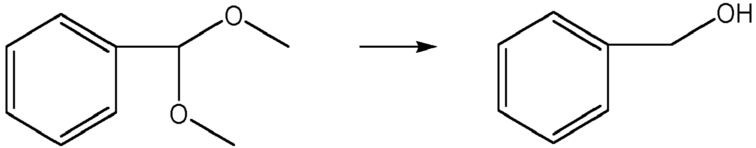

1.5 mL THF, 0.135 g H₂O und 0,42 g (2,72 mmol) Benzaldehyddimethylacetal 1.3 mg (0.18 mol%) RuCl₃ × 3H₂O und 5.2 mg (0.73 mol%) PPh₃ werden in ein Vial gegeben. Es werden 20 bar H₂ aufgepresst und die Reaktion 18 h bei 60 °C durchgeführt. Die GC-Ausbeute beträgt >99%

### F) Phenylacetaldehyddimethylacetal zu 2-Phenylethanol

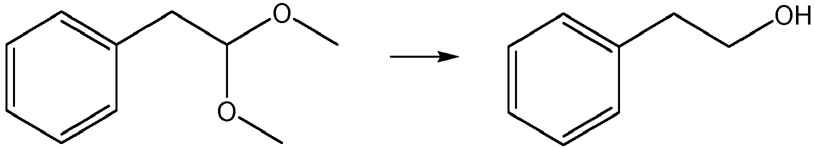

1.5 mL Dioxan, 0.36 g H₂O und 0,42 g (2,5 mmol) Phenylacetaldehyddimethylacetal, 2.6 mg (0.4 mol%) RuCl₃ × 3H₂O und 10.5 mg (1.6 mol%) PPh₃ in ein Vial gegeben. Es werden 20 bar H₂ aufgepresst und die Reaktion 18 h bei 60 °C durchgeführt. Die GC-Ausbeute beträgt >99%.

### G) 4-Methyl-2-octyl-1,3-dioxolan zu 1-Nonanol

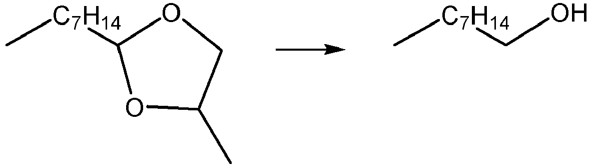

1,4-Dioxan (1.5 mL, absolut), 0.135 mL (7.5 mmol) Wasser und 0.5 mL 4-Methyl-2-octyl-1,3-dioxolan (0.47 g, 2.3 mmol), 2,6 mg (0.4 mol%) RuCl₃ × 3H₂O und 10,5 mg (1.6 mol%) PPh₃ werden in ein Vial gegeben. Es werden 40 bar H₂ aufgepresst und die Reaktion 18 h bei 60 °C durchgeführt. Die GC Ausbeute beträgt 89%.

### H) 2,5-Dimethoxytetrahydrofuran (cis/trans-Gemisch) zu 1,4-Butandiol

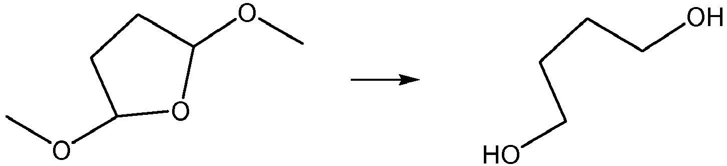

1.5 mL Dioxan, 0.18 mL Wasser und 0,33 g (2.5 mmol) 2,5-Dimethoxytetrahydrofuran (cis/trans-Gemisch), 2.6 mg RuCl₃ × 3H₂O und 10.5 mg (1.6 mol%) PPh₃ werden in ein Vial gegeben. Es werden 20 bar H₂ aufgepresst und die Reaktion 18 h bei 60 °C durchgeführt. Die GC-Ausbeute beträgt 84%.

### I) 3-Ethoxypropionaldehyddiethylacetal zu 3-Ethoxypropanol

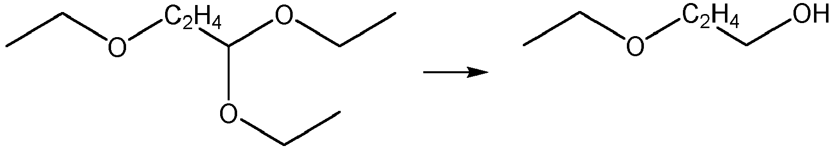

1.5 mL Dioxan, 0.27 g H₂O und 0,451 g (2,56 mmol) Ethoxypropionaldehyddiethylacetal, 2.6 mg (0.39 mol%) RuCl₃ × 3H₂O und 10.5 mg (1.56 mol%) PPh₃ werden in ein Vial gegeben. Es werden 20 bar H₂ aufgepresst und die Reaktion 18 h bei 60 °C durchgeführt. Die GC-Ausbeute beträgt >99%.

### Variation des Katalysatorsystems

### J) Phenylacetaldehyddimethylacetal zu 2-Phenylethanol

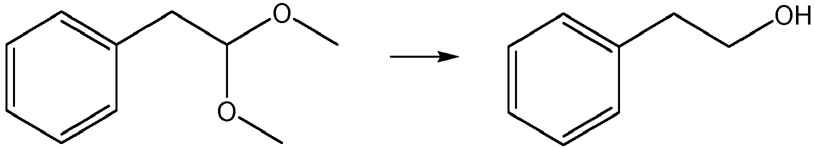

Der feste Katalysator wurde in 8-mL-Vials eingewogen und alle Flüssigkeiten mit der Spritze zugegeben. Das Substrat wurde hierbei zuletzt zugegeben.

Es wurden die folgenden Katalysatorsysteme getestet:
**Kat 1:** 0,4 mol% RuCl₃ × 3 H₂O, 1,6 mol% PPh₃, 0,36 mL Wasser, 1,5 mL Dioxan
**Kat 2:** Ru/C 5%ig (Strem) 44-4065 LOT#:21539500, 50% Wassergehalt, berechnet für 0,67 mol% Metall, 0,17 mL 0,1 M H₂SO₄ (aq), 1,34 mL Wasser, 1,5 mL Methanol
**Kat 3:** Ru/C 5%ig (Johnson-Matthey) Type 622, LOT KS0004, 0,17 mL 0,1 M H₂SO₄ (aq), 1,34 mL Wasser, 1,5 mL Methanol

Als GC-Standard wurde Diglyme, nach der Reaktion zugesetzt.

### Reaktionsbedingungen:

2,5 mmol Substrat, H₂ 20 bar, 60 °C, 5 h.

Die Versuchsergebnisse sind in der nachfolgenden Tabelle aufgelistet:

| Katalysator | Ausbeute |
|---|---|
| Kat 1* | > 98 % |
| Kat 2 | 52 % |
| Kat 3 | 56 % |

| | |
|---|---|
| * erfindungsgemäßes Katalysatorsystem | |

Wie die Versuchsergebnisse zeigen, wird die Aufgabe durch das erfindungsgemäße Verfahren gelöst.

## Patentansprüche

1. Verfahren umfassend die Verfahrensschritte:
a) Vorlegen eines Aldehydacetals gemäß einer der Formeln (**Ia**) bis (**VIa**): 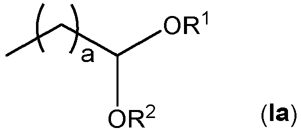 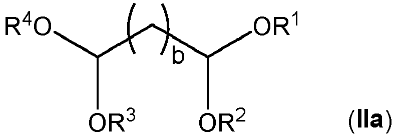 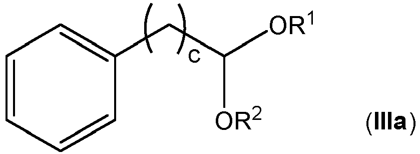 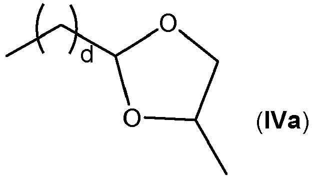 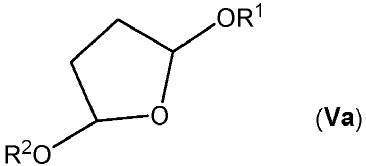 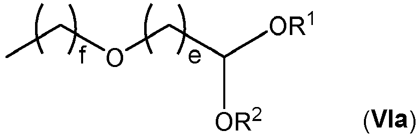
wobei a, c, d, f für eine ganze Zahlen von 0 bis 12 stehen und b, e für eine ganze Zahlen von 1 bis 12 stehen
und R¹, R², R³, R⁴, jeweils unabhängig voneinander, für (C₁-C₁₂)-Alkyl stehen;
b) Zugabe einer Ru-Verbindung, welche zur Ausbildung eines Komplexes befähigt ist und eines Liganden, welcher ein P-Atom aufweist, oder
eines Ru-Liganden-Komplexes, wobei der Ligand des Komplexes ein P-Atom aufweist;
c) Zuführen von H₂;
d) Erhitzen des Reaktionsgemisches aus a) bis c), wobei das Aldehydacetal zu einer Verbindung gemäß Formel (**Ib**) bis (**VIb**) umgesetzt wird:

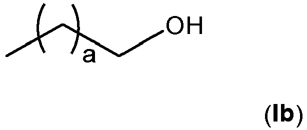

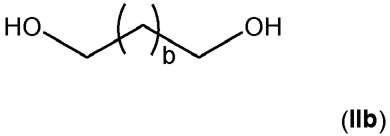

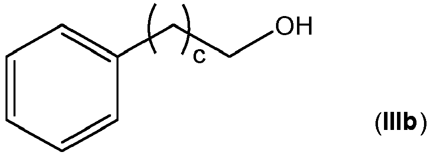

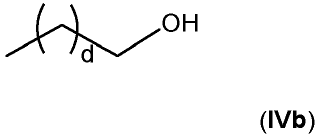

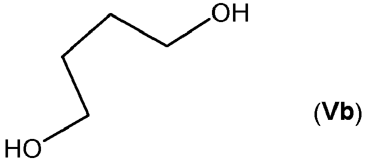

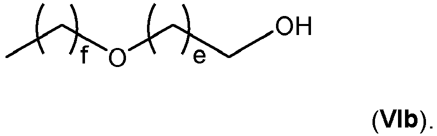

2. Verfahren nach Anspruch 1,
wobei R¹, R² für den gleichen Rest stehen.

3. Verfahren nach einem der Ansprüche 1 oder 2,
wobei R³, R⁴ für den gleichen Rest stehen.

4. Verfahren nach einem der Ansprüche 1 bis 3,
wobei R¹, R², R³, R⁴ für (C₁-C₄)-Alkyl stehen.

5. Verfahren nach einem der Ansprüche 1 bis 4,
wobei die Ru-Verbindung ausgewählt ist aus: RuCl₃ × 3H₂O, [Ru(Cymen)Cl₂]₂, RuBr₃ × 3H₂O, RuI₃, Ru(PPh₃)₃Cl₂.

6. Verfahren nach einem der Ansprüche 1 bis 5,
wobei es sich bei dem Liganden um eine Phosphinliganden handelt.

7. Verfahren nach einem der Ansprüche 1 bis 6,
wobei der Ligand ausgewählt ist aus: PPh₃, 1,4-Bis-(diphenylphosphino)-butan (dppb), 1,1'-Ferrocenediyl-bis(diphenylphosphin) (dppf), Bis-[2-(diphenylphosphino)-phenyl]-ether (dpephos), 1,3-Bis-(diphenylphosphino)-propan (dppp), 4,5-Bis-(diphenylphosphino)-9,9-dimethylxanthen (XantPhos).

8. Verfahren nach einem der Ansprüche 1 bis 7,
wobei das Zuführen von H₂ mit einem Druck erfolgt im Bereich von 0,5 MPa (5 bar) bis 8 MPa (80 bar).

9. Verfahren nach einem der Ansprüche 1 bis 8,
wobei das Erhitzen auf eine Temperatur erfolgt im Bereich von 30 °C bis 100 °C.

10. Verfahren nach einem der Ansprüche 1 bis 9,
umfassend den zusätzlichen Verfahrensschritt c`):
c') Zugabe eines Lösungsmittels.

11. Verfahren nach Anspruch 10,
wobei das Lösungsmittel ausgewählt ist aus: 1,4-Dioxan, Tetrahydrofuran (THF), Wasser.
